## Europäisches Patentamt

## European Patent Office

## Office européen des brevets

(11) Publication number: **0 279 525**
**A1**

# (12) EUROPEAN PATENT APPLICATION

(21) Application number: 88300546.4

(22) Date of filing: 22.01.88

(51) Int. Cl.⁴: **G01N 33/80** , G01N 33/546

(30) Priority: 23.01.87 JP 14758/87

(43) Date of publication of application:
24.08.88 Bulletin 88/34

(84) Designated Contracting States:
DE FR GB

(71) Applicant: SEITETSU KAGAKU CO., LTD.
346-1, Miyanishi Harimacho, Kako-gun
Hyogo-ken 673-01(JP)

(72) Inventor: Kawamura, Masao
18-10 Higashi-Asagirioka
Akashi-shi Hyogo-ken(JP)
Inventor: Akutsu, Seiichi
24-15, Yamate 2-chome
Kakogawa-shi Hyogo-ken(JP)
Inventor: Fukuda, Hirosuke
1044 Imazaike Shikama-ku
Himeji-shi Hyogo-ken(JP)
Inventor: Saga, Kouichi
790-5 Kuchiri Onoe-cho
Kakogawa-shi Hyogo-ken(JP)
Inventor: Okubo, Yasuto
11-18 Taishi 3-chome Ohji-cho
Kitakatsuragi-gun Nara-ken(JP)
Inventor: Seno, Taiko
5-16 Nakahama 3-chome Joto-ku
Osaka-shi Osaka-fu(JP)
Inventor: Morimoto, Shigeru
475-6 Minori Kakogawa-cho
Kakogawa-shi Hyogo-ken(JP)

(74) Representative: Stuart, Ian Alexander et al
MEWBURN ELLIS & CO. 2/3 Cursitor Street
London EC4A 1BQ(GB)

(54) Blood group substance-carrying latex and process for preparing the same.

(57) A blood group substance-carrying latex which comprises latex particles and a blood group substance supported on the particles through chemical bonds such as amide bonds. A process for preparing the latex is also disclosed.

## BLOOD GROUP SUBSTANCE-CARRYING LATEX AND PROCESS FOR PREPARING THE SAME

The present invention relates to a reagent for a so-called "back typing test" in ABO blood grouping and a process for preparing the same. More particularly, it relates to blood group substance-carrying latex particles wherein an ABO blood group substance prepared from, for example, stomach mucosa of various animals or human urine is supported on latex particles and a process for preparing the same.

Usually, ABO blood grouping is carried out by using both "blood group test" wherein the blood group antigen of red blood cells to be tested is detected with standard sera, and so-called "back typing test" wherein anti A and anti B agglutinins in a serum to be tested are detected with known red blood cells of blood groups A and B.

However, there are many problems in such a "back typing test". For example, live red blood cells are often used as the known red cells of blood groups A and B and, therefore, their available period is very short such as for 3 to 4 weeks. Further, in a clinic or the like where fresh blood is hardly obtained, expensive standard blood cells should be purchased. These problems are obstructive to practice of "back typing test" in every blood grouping.

In order to solve these problems, it has been proposed to use blood group substances supported on various carriers instead of using conventional red blood cell reagents. For example, there have been proposed to use blood group A or B substances directly supported on a latex (A. Hagiwara, "Sogo Rinsho", 14, 445 (1965)), on yeast cells (Y. Saneshige, Summary of Lecture, 27th meeting of Transfusion Society of Japan), and on zeolite particles (the present inventors' Japanese Patent Kokai No. 60-40958).

However, in these blood group substance-carrying reagents, the blood group substances are supported on the surfaces of the particles by means of physical adsorption. Therefore, there is such a problem that the substances are liable to remove, which results in low agglutination activity to a serum to be tested. Further, their storage stability and particle dispersion are yet insufficient.

Under these circumstances, the present inventors have studied intensively to develop a stable and cheap reagent for a "back typing test" in blood grouping which can readily detect anti A and anti B agglutinins in a serum to be tested without above problems of conventional reagents. As the result, the present inventors have succeeded in preparation of a blood group substance-carrying latex wherein the blood group substance is supported on the surfaces of latex particles by means of chemical bonds and have found that the latex specifically agglutinates anti A or anti B agglutinin.

Desirably the present invention should provide a blood group substance-carrying latex for a "back typing test" in blood grouping which is stable and cheap, and can readily detect anti A and anti B agglutinins in a serum to be tested.

According to the present invention, there is provided a blood group substance-carrying latex which comprises latex particles and a blood group substance supported on the particles through chemical bonds.

In one aspect of the present invention, a blood group substance is supported on the surfaces of latex particles through amide bonds. More particularly, in this aspect, the desired blood group substance-carrying latex can be prepared by reacting latex particles having carboxyl groups and/or amino groups on their surfaces with a blood group substance having amino and carboxyl groups in the presence of a suitable coupling agent to support the substance on the latex particles through amide bonds thus formed. The latex particles obtained have higher agglutination activity to a serum to be tested and improved storage stability in comparison with a blood group substance-carrying particles prepared by a conventional technique.

In another aspect of the present invention, there is used a protein sensitized latex which is obtained by previously sensitized with protein by means of chemical bonds and/or physical adsorption. Thereby, a much clear image of agglutination can be obtained.

The term "sensitized" used herein means to attach or coat the desired material on the surfaces of the latex particles.

In still another aspect of the present invention, a blood group substance is supported on latex particles through spacers. Thereby, a much clear image of agglutination can be also obtained.

The present invention also provides processes for preparing these blood group substance-carrying latexes.

As naturally occurring ABO blood group substances for human being, not only red blood cells, but also other substances have been found in body fluids or in the bodies of various animals such as swine, bovine and equines. For example, of course, red blood cells of A group man are the A group substance, but A group substances are also present in urine and saliva of A group man and stomach mucosas of swine, goats and the like. Likewise, in addition to red blood cells and body fluids of human being, B group substances are also present in stomach mucosas of equines, frogs, tortoises and the like. Further, the present inventors have found that A and B group substances can be enzymatically synthesized form their precursor, H group substance, in the presence of a sugar-nucleoside (Japanese Patent Kokai No. 60-42655).

The ABO blood group substance used in the present invention is not specifically limited, and there can be used that obtained from any of these suitable sources by isolation and purification on enzymatic synthesis according to a known method. However, apparently, a material in which an A group substance is contaminated with a B group substance or vice versa can not be used from the viewpoint of blood grouping.

In one aspect of the present invention wherein a blood group substance is supported on latex particles through amide bonds, the blood group substance having carboxyl and amino groups is used. In the presence of a coupling agent, the carboxyl group of the blood group substance is subjected to coupling reaction with amino group on the surfaces of latex particles, and the amino group of the blood group substance aree subjected to coupling reaction with carboxyl group of latex particles to form amide bond.

The latex is not specifically limited, and there can be used any latex having carboxyl groups, or amino groups, or both carboxyl and amino groups on the surfaces of particles. Examples of the latex include those of polystyrene, styrene-butadiene copolymer, polymethylmethacrylate, polyvinyltoluene, polyvinyl pyridine, vinyltoluene butylstyrene copolymer, styrene-acrylamide copolymer, styrene-divinylbenzene copolymer, styrene-acrylonitrile copolymer, acrylonitrile-butadinene-styrene copolymer, vinyltoluene-t-butylstyrene copolymer and styrene-vinyltoluene copolymer in which carboxyl and/or amino groups have been introduced. The latex can be prepared by modifying the monomer with amino or carboxyl group and subjecting to a known polymerization such as emulsion polymerization or the like.

Further, in addition to these latexes, there can be also used other latexes originally having carboxyl groups. For example, acrylic acid polymer, methacrylic acid polymer, vinyl acetate-acrylate copolymer, vinyl chloride-acrylate copolymer, styrene-methacrylate copolymer, styrene-acrylate copolymer, methyl methacrylate-methacrylate copolymer, methyl methacrylate-acrylate copolymer, methacrylate-acrylate copolymer and the like can be suitably used. These latexes having carboxyl groups can be used as they are, or further amino groups can be introduced to these latexes.

In order to introduce amino group, the monomer is modified with amino group and subjected to a known polymerization such as emulsion polymerization to obtain the desired latex. These latexes can be used alone or in combination thereof.

Particle size of of the latex to be used is not specifically limited and any particle size can be employed. However, usually, particle size of 0.01 to 20 $\mu$ , preferably 0.1 to 10 $\mu$ are desired. When particle size is less than 0.01 $\mu$ , handling of the latex becomes difficult. On the other hand, when particle size exceeds 20 $\mu$ , particle dispersion becomes inferior. Further, in order to prevent sedimentation and to improve dispersion, it is preferred that a specific gravity of the latex particles are 0.9 to 1.4.

The coupling agent to be used for coupling of amino and carboxyl groups is not specifically limited and any commercially available coupling agent can be used. Examples of the coupling agent include 1,1-carbonyldiimidazole, 1-cyclohexyl-3-(2-morpholinoethyl)carbodiimidemetho-p-toluene sulfonate (hereinafter abbreviated as CMC), N,N-dicyclohexylcarbodiimide, diphenyl phosphorazidate, N-ethoxycarbonyl-2-ethoxy-1,2-dihydroqunoline, 1-ethyl-3-(3-dimethylaminopropyl)carbodiimide hydrochloride (hereinafter abbreviated as EDC), 1-hydroxybenzotriazole, N-hydroxy-5-norbornene-2,3-dicarboxyimide, pivaloyl hydrochloride, N-trifluoroacetylimidazole, N-ethyl-5-phenylisoxazolium-3'-sulfonate (Woodward's Reagent K) and the like.

The amount of the coupling agent to be used is varied depending upon a particular kind of the agent. However, usually, it is preferred to use the coupling agent in an amount of 1 to 100-fold equivalent of carboxyl groups and/or amino groups on the surfaces of the latex particles.

The above latex and the blood group substance which is prepared separately are subjected to a coupling reaction in the presence of the coupling agent to form amide bonds to support the substance on the latex. The coupling reaction is carried out in physiological saline solution, distilled water or a buffer solution adjusted to a suitable pH according to particular kinds of the blood group substance, the latex and the coupling agent used by slowly stirring at 5 to 37°C for 1 to 24 hours.

For example, in the case of using EDC as the coupling agent, 0.01 to 5% (w/v) of latex particles, 0.0001 to 1% (w/v) of a blood group substance and 0.01 to 5% (w/v) of EDC are added to physiological saline solution of pH 4 to 6 or distilled water adjusted to pH 4 to 6 with hydrochloric acid, and the mixture is slowly stirred at room temperature for 1 to 24 hours to support the substance on the latex.

In another aspect of the present invention wherein a latex sensitized with protein is used, firstly, a latex is sensitized with protein. In general, this sensitization can be divided into that through chemical bonds and that by physical adsorption. However, in the present invention. both can be used.

In the case of preparation of a protein sensitized latex by means of chemical bonds, there can be used the above latexes having functional groups such as carboxyl groups and amino groups on the surfaces

3

thereof.

In the case of the preparation of a protein sensitized latex by means of physical adsorption, a latex to be used is not limited to a specific one, and there can be used latexes having no functional group such as polystyrene, polymethacrylate and the like as well as the above latexes having functional groups.

As protein used for sensitization of the latex, there can be used any protein in so far as it is immunologically inactive. Preferred examples of protein include bovine serum albumin (hereinafter abbreviated as BSA), ovalbumin (hereinafter abbreviated as OVA) and the like.

Sensitization of the latex is usually carried out in the presence of a coupling agent. The coupling agent is not specifically limited and the above commercially available coupling agents can be used. Although the amount of coupling agent used in the reaction is varied depending upon a particular kind of the coupling agent used. Usually, the coupling agent is used in an amount of 1 to 100-fold equivalent of carboxyl groups and/or amino groups on the surfaces of the latex.

That is, in the case of the sensitization of the latex with protein by means of chemical bonds, the latex and protein are subjected to a coupling reaction in the presence of a coupling agent to form chemical bonds between the latex and protein. The coupling reaction can be carried out in water, physiological saline solution or a buffer solution adjusted to a suitable pH according to particular reaction conditions by slowly stirring at 5 to 37°C for 1 to 24 hours.

For example, in the case of using EDC as the coupling agent, 0.01 to 0.5% (w/v) of latex particles, 0.001 to 1% (w/v) of BSA and 0.01 to 5% (w/v) of EDC are slowly stirred in physiological saline solution of pH 4 to 6 or water of pH 4 to 6 at room temperature for 1 to 24 hours to couple BSA to the latex. After coupling, the latex is thoroughly washed with water or physiological saline solution and then the latex is dispersed in the same solution.

In the case of the sensitization of the latex with protein by means of physical adsorption, firstly, protein is dissolved in a buffer solution of a suitable pH. Then, latex particles are dispersed in the solution and the mixture is slowly stirred at 20 to 37°C for 1 to 24 hours to adsorb protein to the latex. After adsorption, un-adsorbed protein is thoroughly washed and the protein sensitized latex is separated and dispersed in physiological saline solution or water.

Then, a blood group substance is introduced to support the substance on the protein sensitized latex thus prepared.

As a method for supporting the substance on the latex, in addition to the above chemical method using the coupling agent, there can be also used the following method wherein the substance is supported on the latex through chromium ion in the presence of sodium deoxycholate (hereinafter abbreviated as DOC).

In general, a trivalent metallic ion such as chromium ion has such property that it is tightly bound to protein. By utilizing this property, an antigen or antibody is coated on red blood cells in the presence of an aqueous solution of chromic chloride ($CrCl_3$) and they are used for detection of a corresponding antibody or antigen (Gold and Fundenberg, "J. Immunology", 1967, 99, p 859-866). Further, it has been reported that, when an antigen or antibody is supported in the presence of DOC, a larger amount of the antigen or antibody can be supported thereon and agglutination to an agglutinin is improved (Michael Steinitz and Sara Tamir, "J. Clinical Laboratory Immunology", 1985 18, p 195-198).

However, there can not be found any report of supporting a blood group substance on a latex by using this method.

Thus, in this aspect of the present invention, a blood group substance can be supported on, for example, a BSA sensitized latex through $CrCl_3$ in the presence of DOC. That is, a blood group substance is dissolved in an aqueous solution of DOC. The above protein sensitized latex is dispersed in the solution and then a $CrCl_3$ solution is added. These components are used in the concentrations of 0.01 to 0.5% of DOC, 0.001 to 0.5% of the blood group substance and 5 to 100 mM of $CrCl_3$. Then, stirring is continued slowly at 4 to 25°C for 1 to 60 minutes to support the blood group substance on the protein sensitized latex and the resulting latex particles are thoroughly washed with physiological saline solution or water. The latex particles are dispersed in a suitable buffer solution, for example, 50 mM phosphate buffer solution of pH 8 (hereinafter abbreviated as PB) or 50 mM phosphate buffer solution of pH 8 containing 0.85% of NaCl (hereinafter abbreviated as PBS) to obtain the desired blood group substance-carrying latex.

The chemical method for supporting a blood group substance on the latex using the above coupling agent such as EDC or CMC can be carried out according to the same manner as the above sensitization of protein to the latex.

In the case of supporting the blood group substance on the latex particles through spacers according to the present invention, the latexes having functional groups for binding spacers are used. For example, in order to bind spacers by means of covalent bonds, there can be used the above latexes having functional groups such as carboxyl groups, amino groups and the like.

As the spacer, there can be used that having functional groups such as amino group and/or carboxyl group at both ends thereof.

Representative examples of the spacer include amino acids having 4 to 10 carbon atoms, alkylene diamines having 3 to 8 carbon atoms, polyalkylene polyamines having 8 to 12 carbon atoms, polyethylene polyamines having 4 to 6 carbon atoms and dicarboxylic acids having 9 to 14 carbon atoms.

As amino acids, there can be used, for example, 6-amino-n-caproic acid, γ-amino-n-butyric acid, ω-amino-caprylic acid, lysine, arginine and glutamic acid. As alkylene diamines, there can be used, for example, trimethylene diamine, heptamethylene diamine and octamethylene diamine. As polyalkylene polyamines, there can be used, for example, thermospermine and spermine. As ethylene amine, there can be used, for example, pentaethylene hexamine. As dicarboxylic acids, there can be used, for example, brassylic acid.

Particularly, good results can be obtained by using 6-amino-n-caproic acid, γ-amino-n-butyric acid, ω-aminocaprylic acid, trimethylene diamine, heptamethylene diamine or octamethylene diamine.

In order to bind the spacer to the latex, usually, they are subjected to a coupling reaction in the presence of a coupling agent in water, physiological saline solution or a buffer solution adjusted to a suitable pH.

The coupling agent is not specifically limited and the above commercially available coupling agents can be used. Although the amount of the coupling agent is varied depending upon a particular kind thereof, usually, it is used in an amount of 10 to 100-fold equivalent of carboxyl groups and/or amino groups of the surfaces of the latex.

In the present invention, the spacers are bound to latex and the spacer sensitized latex thus obtained is used to support the blood group substance thereon. The binding and supporting the blood group substance can be carried out according to the same manner as described in the sensitization of the latex with the spacers.

Optionally, the blood group substance-carrying latex particles of the present invention thus prepared can be stabilized by addition of an immunologically inactive compound, for example, 0.01 to 1% (w/v) of BSA to prevent non-specific agglutination or spontaneous agglutination of the particles. Thereby, good results can be obtained. Further, as a preservative, sodium azide ($NaN_3$) can be added in the concentration of 0.05 to 0.5%. The blood group substance-carrying latex thus obtained can be stored stably for more than one year.

The blood group substance-carrying latex of the present invention is used for a so-called "back typing test" in ABO blood grouping and anti A and anti B agglutinins in a serum can be readily detected according to a slide method. That is, each one drop of a serum to be tested is dropped at two parts of a slide glass plate. A predetermined amount of an A group substance-carrying latex is added dropwise to the serum at one part and a B group substance-carrying latex is added dropwise to the serum at the other part. They are slowly swirled. By observing whether agglutination is present or not, blood grouping can be readily carried out within several minutes.

The following Examples and Experiments further illustrate the present invention in detail but are not to be construed to limit the scope thereof. In Examples and Experiments, all concentrations are w/v unless otherwise stated.

Water used in the reactions were distilled water and adjustment of acidity was effected by hydrochloric acid.

Example 1

(I) Preparation of A group substance

Swine stomach mucosa (320 g) was suspended in water (300 ml) and homogenized with a mixer. The homogenate was centrifuged. The supernatant was adjusted to pH 2 with hydrochloric acid and treated with pepsin at 37°C for 4 days. After digestion with pepsin, the precipitate was removed by centrifugation and ethanol was added to the supernatant to bring 70% saturation. The mixture was allowed to stand for one day and night. The precipitate formed was collected and washed with ethanol and ether and dried to obtain a crude A group substance (3 g). The crude substance obtained was dissolved in 10 mM phosphate buffer solution (pH 7.0) and subjected to gel filtration with Sepharose 4B resin (manufactured by Pharmacia, Sweden) to obtain the purified A.

(II) Preparation of A group substance-carrying latex

The A group substance obtained in the above (I) was added to the latex, "Immutex G 0401" (manufactured by Nippon Gosei Gomu Kabushiki Kaisha, Japan; average particle size: 1.096 μ ; surface charge: 0.087 meqCOO⁻/g) suspended in water. To the mixture was added the coupling agent, EDC and pH thereof was adjusted to 4.5 with hydrochloric acid. The concentrations of the components in the mixture were 2.5% of the latex, 0.015% of the A group substance and 1% of EDC. Then, stirring was continued slowly at 20°C for 2 hours to form amide bonds by coupling reaction. The latex particles were collected by centrifugation. Then, the particles were thoroughly washed with water acidified with hydrochloric acid (pH 4.5) and aqueous 1% BSA solution (pH 4.5) was added to adjust the concentration of the latex particles to 1%. The mixture was further stirred at 20°C for 2 hours to stabilize the latex particles. Again, the mixture was centrifuged to collect the particles. The latex particles were thoroughly washed with 50 mM tris buffer solution (tris(hydroxymethyl)aminomethane hydrochloride buffer solution, pH 8.0) and the particles were suspended in the same buffer solution to obtain a suspension containing the desired A group substance-carrying latex in the concentration of 0.2%.

When anti A serum was added to this latex, agglutination reaction occurred. However, when anti B serum was used, no agglutination was observed.

Example 2

The A group substance obtained in the above Example 1 (I) was added to the latex, "Immutex G 0101" (manufactured by Nippon Gosei Gomu Kabushiki Kaisha, Japan; average particle size: 0.7 μ ; surface charge: 0.03 meqCOO⁻/g) suspended in water. To the mixture was added the coupling agent, EDC and pH thereof was adjusted to 4.5 with hydrochloric acid. The concentration of the component in the mixture were 5.0% of the latex, 0.01% of the A group substance and 1% of EDC. Then, stirring was continued slowly at 20°C for 4 hours to form amide bonds by coupling reaction. The latex particles were collected by centrifugation. Then, the particles were thoroughly washed with water acidified with hydrochloric acid (pH 4.5) and then washed with 50 mM tris buffer solution (pH 8.0) and the particles were suspended in the same buffer solution to obtain a suspension containing the desired A group substance-carrying latex in the concentration of 0.2%.

When anti A serum was added to this latex, agglutination reaction occurred. However, when anti B serum was used, no agglutination was observed.

Example 3

To 50 mM phosphate buffer solution (pH 8.5) in which the latex, "Immutex G 0401" was dispersed in the concentration of 3% was added the coupling agent, "Woodward's Reagent K" in the concentration of 0.2%. The mixture was slowly stirred at 5°C for 3 hours to contact both components and centrifuged to collect latex particles. The particles were washed with water. The particles were added to the A group substance obtained in Example 1 (I) in 50 mM phosphate buffer solution (pH 5.5) and stirring was continued slowly at 5°C for 3 hours to form amide bonds by coupling reaction. The concentrations of the components in the reaction mixture were 3% of the latex and 0.5% of the A group substance. After completion of the reaction, the latex particles were collected by centrifugation. Then, the particles were thoroughly washed with 50 mM phosphate buffer solution (pH 5.5) and the mixture was centrifuged to collect the particles. The latex particles were suspended in 50 mM tris buffer solution (pH 8.0) containing as a preservative sodium azide (0.01%) to obtain a suspension containing the desired A group substance-carrying latex in the concentration of 0.2%.

When anti A serum was added to this latex, agglutination reaction occurred. However, when anti B serum was used, no agglutination was observed.

Example 4

The latex, "Immutex G 0401" was suspended in water and to the suspension were added the A group substance obtained in Example 1 (I) and CMC. The pH was adjusted to 5.0. The concentrations of the components were 2.5% of the latex, 0.015% of the A group substance and 0.5% of CMC. The mixture was slowly stirred at 20°C for 5 hours to proceed coupling reaction and the latex particles were collected by centrifugation. The particles were thoroughly washed with water of pH 5 and then with 50 mM tris buffer solution (pH 8.0). The latex particles were suspended in the same buffer to obtain a suspension containing the desired A group substance-carrying latex in the concentration of 0.2%.

When anti A serum was added to this latex, agglutination reaction occurred. However, when anti B serum was used, no agglutination was observed.

Example 5

The latex, "POLYBEAD" (fine particles having carboxyl groups on the surfaces there of manufactured by Polyscience Inc., Warrington, U.S.A.; average particle size: 5.2 $\mu$ ; surface charge: 0.12 meqCOO$^-$/g) was suspended in water and to the suspension were added the A group substance obtained in Example 1 (I) and EDC. The pH was adjusted to 4.5. The concentrations of the components were 5% of the latex, 0.002% of the A group substance and 2% of EDC. The mixture was slowly stirred at 20°C for 2 hours to form amide bonds by coupling reaction and the latex particles were collected by centrifugation. The particles were thoroughly washed with water acidified with hydrochloric acid to pH 4.5 and then with 50 mM tris buffer solution (pH 8.0). The latex particles were suspended in the same buffer to obtain a suspension containing the desired A group substance-carrying latex in the concentration of 0.2%.

When anti A serum was added to this latex, agglutination reaction occurred. However, when anti B serum was used, no agglutination was observed.

Example 6

The latex, "POLYBEAD" (fine particles having amino groups on the surfaces thereof manufactured by Polyscience Inc., Warrington, U.S.A.; average particle size: 3.0 $\mu$ ; amino group content: 0.2 $\mu$mol of amino group/4 mg of polymer) was suspended in water and to the suspension were added the A group substance obtained in Example 1 (I) and EDC. The pH was adjusted to 4.5. The concentrations of the components were 2.5% of the latex, 0.015% of the A group substance and 1% of EDC. The mixture was slowly stirred at 20°C for 2 hours to proceed coupling reaction and the latex particles were collected by centrifugation. The particles were thoroughly washed with water acidified with hydrochloric acid to pH 4.5 and then with 50 mM tris buffer solution (pH 8.0). The latex particles were suspended in the same buffer to obtain a suspension containing the desired A group substance-carrying latex in the concentration of 0.2%.

When anti A serum was added to this latex, agglutination reaction occurred. However, when anti B serum was used, no agglutination was observed.

Example 7

(I) Preparation of B group substance

According to the same manner as described in Example 1 (I), a crude B group substance (1.5 g) was obtained from equine stomach mucosa (200 g). Further, the crude substance was subjected to gel filtration with Sepharose 4 B resin to obtain the purified B group substance.

(II) Preparation of B group substance-carrying latex

The latex, "Immutex G 0401" was suspended in water and to the suspension were added the B group substance obtained in (I) and EDC. The pH was adjusted to 4.5. The concentrations of the components were 5% of the latex, 0.002% of the B group substance and 2% of EDC. The mixture was slowly stirred at 20°C for 2 hours to form amide bonds by coupling reaction and the latex particles were collected by centrifugation. The particles were thoroughly washed with water acidified with hydrochloric acid to pH 4.5 and aqueous 1% BSA solution (pH 4.5) was added to adjust the concentration of the latex to 1%. The mixture was stirred slowly at 20°C for 2 hours to stabilize the latex particles. The latex particles were collected by centrifugation. The particles were thoroughly washed with 50 mM tris buffer solution (pH 8.0).

The latex particles were suspended in the same buffer to obtain a suspension containing the desired B group substance-carrying latex in the concentration of 0.2%.

When anti B serum was added to this latex, agglutination reaction occurred. However, when anti A serum was used, no agglutination was observed.

Example 8

The latex, "Immutex G 0101" was suspended in water and to the suspension were added the B group substance obtained in Example 7 (I) and EDC. The pH was adjusted to 4.5. The concentrations of the components were 5% of the latex, 0.01% of the B group substance and 1% of EDC. The mixture was slowly stirred at 20°C for 2 hours to proceed coupling reaction and the latex particles were collected by centrifugation. The particles were thoroughly washed with water at pH 4.5 and then with 50 mM tris buffer solution (pH 8.0). The latex particles were suspended in the same buffer to obtain a suspension containing the desired B group substance-carrying latex in the concentration of 0.2%.

When anti B serum was added to this latex, agglutination reaction occurred. However, when anti A serum was used, no agglutination was observed.

Example 9

The latex, "POLYBEAD" (fine particles having carboxyl groups on the surfaces thereof manufactured by Polyscience Inc., Warrington, U.S.A.; average particle size: 5.2 $\mu$ ; surface charge: 0.12 meqCOO$^-$/g) was suspended in water and to the suspension were added the B group substance obtained in Example 7 (I) and EDC. The pH was adjusted to 4.5. The concentrations of the components were 2.5% of the latex, 0.01% of the B group substance and 2% of EDC. The mixture was slowly stirred at 4°C for 1 hour to proceed coupling reaction and the latex particles were collected by centrifugation. The particles were thoroughly washed with water at pH 4.5 and then with 50 mM tris buffer solution (pH 8.0). The latex particles were suspended in the same buffer to obtain a suspension containing the desired B group substance-carrying latex in the concentration of 0.2%.

When anti B serum was added to this latex, agglutination reaction occurred. However, when anti A serum was used, no agglutination was observed.

Example 10

The latex, "POLYBEAD" (fine particles having amino groups on the surfaces thereof manufactured by Polyscience Inc., Warrington, U.S.A.; particle size: 1.0 $\mu$ ; amino group content: 0.2 $\mu$mol amino group/1 mg polymer) was suspended in water and to the suspension were added the B group substance obtained in Example 7 (I) and EDC. The pH was adjusted to 4.5. The concentrations of the components were 2.5% of the latex, 0.015 % of the B group substance and 1% of EDC. The mixture was slowly stirred at 20°C for 2 hours to proceed coupling reaction and the latex particles were collected by centrifugation. The particles were thoroughly washed with water acidified with hydrochloric acid to pH 4.5 and then with 50 mM tris buffer solution (pH 8.0). The latex particles were suspended in the same buffer to obtain a suspension containing the desired B group substance-carrying latex in the concentration of 0.2%.

When anti B serum was added to this latex, agglutination reaction occurred. However, when anti A serum was used, no agglutination was observed.

Experiment 1

Blood grouping according to slide method

Blood grouping was carried out by using the A group substance-carrying latex obtained in Example 1 and the B group substance-carrying latex obtained in Example 7 according to slide method. The test procedure and the results are as follows.

Each one drop of a serum to be tested was dropped at two parts of a slide glass. Then, one drop of the A group substance-carrying latex was added dropwise at one part and one drop of the B group substance-carrying latex was added dropwise to the other part. The slide glass was slowly swirled for several minutes and the presence of agglutination was judged.

The resuls obtained from 20 sera are shown in Table 1.

In Table 1, the symbols represents as follows.

+: agglutination

-: no agglutination

## Table 1

| Sera No. | Agglutination | | Judgment | Blood group test |
|---|---|---|---|---|
| | A group substance carrying latex | B group substance carrying latex | | |
| 1 | + | + | O | O |
| 2 | - | + | A | A |
| 3 | + | + | O | O |
| 4 | - | - | AB | AB |
| 5 | - | + | A | A |
| 6 | + | + | O | O |
| 7 | + | + | O | O |
| 8 | - | + | A | A |
| 9 | + | - | B | B |
| 10 | - | + | A | A |
| 11 | - | - | AB | AB |
| 12 | + | - | B | B |
| 13 | + | + | O | O |
| 14 | - | + | A | A |
| 15 | - | + | A | A |
| 16 | + | - | B | B |
| 17 | - | + | A | A |
| 18 | - | - | AB | AB |
| 19 | + | + | O | O |
| 20 | - | + | A | A |

As seen from Table 1, the test results obtained by using the blood group substance-carrying latex of the present invention are completely corresponding to those of the blood group test.

Further, according to the same slide method as described above, blood grouping of various sera was carried out by using various combinations of the A group substance-carrying latexes obtained in Examples 2 to 6 and the B group substance-carrying latexes obtained in Examples 8 to 10. The results were completely corresponding to those of the blood group test.

Example 11

(I) Preparation of BSA sensitized latex

BSA was added to the latex, "Immutex G 0401" suspended in water and to the suspension was added the coupling agent, EDC. The mixture was adjusted to pH 4.5 with hydrochloric acid. The concentrations of the components in the mixture were 2.5% of the latex, 0.05% of BSA and 1% of EDC.

Then, the mixture was stirred slowly at 20°C for 1 hour to form amide bonds by coupling reaction. The latex particles were collected by centrifugation. The particles were thoroughly washed with water acidified with hydrochloric acid to pH 4.5 and then with physiological saline solution. The particles were suspended in a physiological saline solution in the concentration of 10% to obtain a suspension of the desired BSA sensitized latex.

(II) Preparation of A group substance-carrying latex

The A group substance obtained in Example 1 (I) was dissolved in aqueous 0.3% DOC solution and the solution was added to the BSA sensitized latex obtained ins the above (I). Further, aqueous 50 mM $CrCl_3$ solution was added. The concentrations of the components in the mixture were 2% of the BSA sensitized latex, 0.04% of the A group substance, 0.12% of DOC and 20 mM of $CrCl_3$. Then, stirring was continued slowly at 20°C for 5 minutes to support the A group substance on the BSA sensitized latex through chromium ion. The latex particles were collected by centrifugation. Then, the particles were thoroughly washed with physiological saline solution and aqueous 1% BSA solution was added to adjust the concentration of the latex particles to 1%. The mixture was further stirred at 20°C for 1 hours to stabilize the latex particles. Again, the mixture was centrifuged to collect the particles. The latex particles were thoroughly washed with 50 mM tris buffer solution (pH 8.0) and the particles were suspended in the same buffer solution to obtain a suspension containing the desired A group substance-carrying latex in the concentration of 0.2%.

When anti A serum was added to this latex, agglutination reaction occurred. However, when anti B serum was used, no agglutination was observed.

Example 12

The A group substance obtained in Example 1 (I) was added to the BSA sensitized latex dispersion obtained in Example 11 (I) and to the mixture was further added CMC. The mixture was adjusted to pH 4.5 with hydrochloric acid. The concentrations of the components in the mixture were 5% of the BSA sensitized latex, 0.01% of the A group substance and 1% of CMC. The mixture was slowly stirred at 20°C for 1 hour to form amide bonds between BSA and the A group substance by coupling reaction and the latex particles were collected by centrifugation. The particles were thoroughly washed with water acidified with hydrochloric acid to pH 4.5 and then with 50 mM tris buffer solution (pH 8.0). The latex particles were suspended in the same buffer solution in the concentration of 0.2% to obtain a suspension of the desired A group substance-carrying latex.

When anti A serum was added to this latex, agglutination reaction occurred. However, when anti B serum was used, no agglutination was observed.

Example 13

(I) Preparation of BSA sensitized latex

The the latex, "Immutex G 2401" (manufactured by Nippon Gosei Gomu Kabushiki Kaisha, Japan; average particle size: 0.4 $\mu$ ; no functional group) was added BSA dissolved in 50 mM PBS (pH 6.0). The concentrations of the components in the mixture were 0.5% of the latex and 1% of BSA. Then, the mixture was stirred slowly at 37°C for 2 hours to sensitize the latex with BSA by physical adsorption. The latex particles were collected by centrifugation. The particles were thoroughly washed with physiological saline solution and suspended in the solution to obtain a suspension of the BSA sensitized latex.

(II) Preparation of A group substance-carrying latex

By using $CrCl_3$ according to the same manner as described in Example 11 (II), the A group substance was supported on the BSA sensitized latex obtained in the above (I).

When anti A serum was added to the resulting desired A group substance-carrying latex, agglutination occcurred. However, no agglutination was observed by using anti B serum.

## Example 14

The A group substance obtained in Example 1 (I) was added to the disperson of the BSA sensitized latex obtained in Example 13 (I). According to the same manner as described in Example 12, coupling reaction was carried out by using EDC to obtain the desired A group substance-carrying latex.

When anti A serum was added to the latex, agglutination occurred. However, no agglutination was observed by using anti B serum.

## Example 15

(I) Preparation of BSA sensitized latex

According to the same manner as described in Example 11 (I), BSA was coupled to the latex "POLYBEAD" of fine particles having carboxyl groups on the surfaces thereof manufactured Polyscience Inc., Warrington, U.S.A. (average particle size: 5.2 $\mu$ ; surface charge: 0.12 meqCOO$^-$/g) by using CMC to obtain the desired BSA sensitized latex.

(II) Preparation of A group substance-carrying latex

According to the same manner as described in Example 11 (II), the A group substance obtained in Example 1 (I) was added to the dispersion of BSA sensitized latex obtained in the above (I) to support the A group substance on the BSA sensitized latex through $CrCl_3$.

When anti A serum was added to the latex, agglutination occurred. However, no agglutination was observed by using anti B serum.

## Example 16

According to the same manner as described in Example 15, an A group substance-carrying latex was obtained except that the latex "POLYBEAD" of fine particles having amino groups on the surfaces thereof manufactured by Polyscience Inc., Warrington, U.S.A. (average particle size: 3.0 $\mu$ ; amino group content: 0.2 $\mu$mol amino group/1 mg of polymer) was used.

When anti A serum was added to the latex, agglutination occurred. However, no agglutination was observed by using anti B serum.

## Example 17

Preparation of B group substance-carrying latex

According to the same manner as described in Example 11 (II), the B group substance obtained in Example 7 (I) was added to the BSA sensitized latex obtained in Example 11 (I) to support the B group substance on the BSA sensitized latex through CrCl₃.

When anti B serum was added to the latex, agglutination occurred. However, no agglutination was observed by using anti A serum.

Example 18

According to the same manner as described in Example 2, the B group substance obtained in Example 7 (I) was added to the BSA sensitized latex obtained in Example 11 (I) and coupling reaction was carried out by using CMC to obtain the desired B group substance-carrying latex.

When anti B serum was added to the latex, agglutination occurred. However, no agglutination was observed by using anti A serum.

Example 19

According to the same manner as described in Example 11 (II), the B group substance obtained in Example 7 (I) was added to the BSA sensitized latex obtained in Example 13 (I) to support the B group substance on the BSA sensitized latex through CrCl₃.

When anti B serum was added to the latex, agglutination occurred. However, no agglutination was observed by using anti A serum.

Example 20

(I) Preparation of OVA sensitized latex

According to the same manner as described in Example 1 (II), OVA was coupled to the latex, "Immutex G 0101 (manufactured by Nippon Gosei Gomu Kabushiki Kaisha, Japan; average particle size: 0.7 $\mu$ ; surface charge: 0s.03 meqCOO⁻/g) by using EDC to obtain a OVA sensitized latex.

(II) Preparation of B group substance-carrying latex

According to the same manner as described in Example 11 (II), the B group substance obtained in Example 7 (I) was added to the dispersion of OVA sensitized latex obtained in the above (I) to support the B group substance on the OVA sensitized latex through CrCl₃ to obtain the desired B group substance-carrying latex.

When anti B serum was added to the latex, agglutination occurred. However, no agglutination was observed by using anti A serum.

Example 21

(I) Preparation of BSA sensitized latex

According to the same manner as described in Example 13, the latex was sensitized with BSA by physical adsorption to obtain the desired BSA sensitized latex except that polystyrene fine particles manufactured by Polyscience Inc., Warrington, U.S.A. (average particle size: 5.6 $\mu$; no functional group) was used instead of "Immutex G 2401".

(II) Preparation of B group substance-carrying latex

According to the same manner as described in Example 11 (II), the B group substance obtained in Example 7 (I) was added to the BSA sensitized latex obtained in above (I) to support the B group substance on the BSA sensitized latex through $CrCl_3$ to obtain the desired B group substance-carrying latex.

When anti B serum was added to the latex, agglutination occurred. However, no agglutination was observed by using anti A serum.

Example 22

According to the same manner as described in Example 12, the B group substance obtained in Example 7 (I) was coupled with the BSA sensitized latex obtained in Example 13 (I) by using CMC to obtain the desired B group substance-carrying latex.

When anti B serum was added to the latex, agglutination occurred. However, no agglutination was observed by using anti A serum.

Experiment 2

Blood grouping according to slide method

According to the same manner as described above, blood grouping was carried out by using the A group substance-carrying latex obtained in Example 11 and the B group substance-carrying latex obtained in Example 17 according to slide method. The results obtained from 25 sera are shown in Table 2.

Table 2

| Sera No. | Agglutination | | Judgment | Blood group test |
|---|---|---|---|---|
| | A group substance carrying latex | B group substance carrying latex | | |
| 1 | - | - | AB | AB |
| 2 | - | + | A | A |
| 3 | + | + | 0 | 0 |
| 4 | - | + | A | A |
| 5 | + | - | B | B |
| 6 | - | - | AB | AB |
| 7 | + | + | 0 | 0 |
| 8 | - | + | A | A |
| 9 | - | + | A | A |
| 10 | + | + | 0 | 0 |
| 11 | + | - | B | B |
| 12 | - | + | A | A |
| 13 | + | - | B | B |

## Table 2 (continued)

| Sera No. | Agglutination | | Judgment | Blood group test |
|---|---|---|---|---|
| | A group substance carrying latex | B group substance carrying latex | | |
| 14 | − | + | A | A |
| 15 | + | + | O | O |
| 16 | − | + | A | A |
| 17 | + | + | O | O |
| 18 | + | − | B | B |
| 19 | − | + | A | A |
| 20 | + | + | O | O |
| 21 | + | − | B | B |
| 22 | + | + | O | O |
| 23 | − | − | AB | AB |
| 24 | − | + | A | A |
| 25 | − | + | A | A |

As seen from Table 2, the test results obtained by using the blood group substance-carrying latex of the present invention are completely corresponding to those of the blood group test.

Further, according to the same slide method as described above, blood grouping of various sera was carried out by using various combinations of the A group substance-carrying latexes obtained in Examples 12 to 16 and the B group substance-carrying latexes obtained in Examples 18 to 22. The results were completely corresponding to those of the blood group test.

Example 23

(I) Preparation of spacer sensitized latex

6-Amino-n-caproic acid was added to the latex, "Immutex G 0401" suspended in water and the coupling agent, EDC was added to the mixture. The mixture was adjusted to pH 6 with hydrochloric acid. The concentrations of the components in the mixture were 2.5% of the latex, 0.5% of 6-amino-n-caproic acid and 1% of EDC. The mixture was stirred slowly at 20°C for 1 hour to proceed coupling reaction and the latex particles were collected by centrifugation. The particles were thoroughly washed with physiological saline solution and suspended in the same solution in the concentration of 10% to obtain a suspension of the desired spacer sensitized latex.

(II) Preparation of A group substance-carrying latex

The A group substance obtained in Example 1 (II) was added to the suspension of the spacer sensitized latex obtained in the above (I). To the mixture was added the coupling agent, EDC and pH thereof was adjusted to 6 with hydrochloric acid. The concentration of the components in the mixture were 5% of the spacer sensitized latex, 0.01% of the A group substance and 1% of EDC. Then, stirring was continued slowly at 20°C for 1 hours to bind the A group substance to the ends of the spacers by coupling reaction. The latex particles were collected by centrifugation. Then, the particles were thoroughly washed with physiological saline solution and further with 50 mM tris buffer solution (pH 8.0). The particles were suspended in the same buffer solution in the concentration of 0.2% to obtain a suspension of the desired A group substance-carrying latex.

When anti A serum was added to this latex, agglutination reaction occurred. However, when anti B serum was used, no agglutination was observed.

## Example 24

According to the same manner as described in Example 23 (II), the A group substance prepared in Example 1 (I) was supported on the spacer sensitized latex prepared in Example 23 (I)except that CMC was used as the coupling agent instead of EDC.

When anti A serum was added to the resulting latex, agglutination reaction occurred. However, when anti B serum was used, no agglutination was observed.

## Example 25

(I) Preparation of spacer sensitized latex

γ-Amino-n-butyric acid was dissolved in 0.01M borate buffer solution (pH 8.1). The latex, "Immutex G 0101" was suspended in the solution and further, the coupling agent, EDC was added to the suspension. The concentrations of the components in the mixture were 2.5% of the latex, 1% of γ-amino-n-butyric acid and 1% of EDC. The mixture was stirred slowly at 20°C for 1 hour to proceed coupling reaction and the latex particles were collected by centrifugation. The particles were thorougly washed with 0.01 M borate buffer solution (pH 8.1) and suspended in the same solution in the concentration of 10% to obtain a suspension of the desired spacer sensitized latex.

(II) Preparation of A group substance-carrying latex

The A group substance obtained in Example 1 (I) was dissolved in 0.01 M borate solution (pH 8.1) and added to the dispersion of the spacer sensitized latex obtained in the above (I). To the mixture was added the coupling agent, EDC and stirred slowly at 20°C for 1 hour to proceed coupling reaction. The concentration of the components in the mixture were 5% of the spacer sensitized latex, 0.01% of the A group substance and 1% of EDC. After completion of coupling, the latex particles were collected by centrifugation. Then the particles were thoroughly washed with physiological saline solution and further with 50 mM tris buffer solution (pH 8.0). The particles were suspended in the same buffer solution in the concentration of 0.2% to obtain a suspension of the desired A group substance-carrying latex.

When anti A serum was added to this latex, agglutination reaction occurred. However, when anti B serum was used, no agglutination was observed.

## Example 26

(I) Preparation of spacer sensitized latex

According to the same manner as described in Example 23 (I), a spacer sensitized latex was prepared except that ω-aminocaprylic acid was used as the spacer and a latex "POLYBEAD" of fine particles having amino groups on the surface thereof manufactured by Polyscience Inc., Warrington, U.S.A. (average particle size: 3.0 μ ; amino group content: 0.2 μmol amino group/1 mg of polymer) was used as the latex.

(II) Preparation of A group substance-carrying latex.

According to the same manner as described in Example 23 (II), the A group substance obtained in Example 1 (I) was supported on the spacer sensitized latex obtained in the above (I).

When anti A serum was added to this latex, agglutination reaction occurred. However, when anti B serum was used, no agglutination was observed.

Example 27

(I) Preparation of spacer sensitized latex

According to the same manner as described in Example 25 (I), a spacer sensitized latex was prepared except that octamethylene diamine was used as the spacer and a latex "POLYBEAD" of fine particles having carboxyl groups on the surfaces thereof manufactured by Polyscience Inc., Warrington, U.S.A. (average particle size: 5.2 μ ; surface charge: 0.12 meqCOO$^-$/g) was used as the latex.

(II) Preparation of A group substance-carrying latex

According to the same manner as described in Example 25 (II), the A group substance obtained in Example 1 (I) was supported on the spacer sensitized latex obtained in the above (I).

When anti A serum was added to this latex, agglutination reaction occurred. However, when anti B serum was used, no agglutination was observed.

Example 28

(I) Preparation of spacer sensitized latex

According to the same manner as described in Example 25 (I), a spacer sensitized latex was prepared except that trimethylene diamine was used as the spacer and, as the latex , "DRS-01" (manufactured by Nippon Gosei Gomu Kabushiki Kaisha, Japan; particle size: 3 μ ) was used.

(II) Preparation of A group substance-carrying latex

According to the same manner as described in Example 25 (II), the A group substance obtained in Example 1 (I) was supported on the spacer sensitized latex obtained in the above (I).

When anti A serum was added to this latex, agglutination reaction occurred. However, when anti B serum was used, no agglutination was observed.

Example 29

(I) Preparation of spacer sensitized latex

According to the same manner as described in Example 25 (I), a spacer sensitized latex was prepared except that heptamethylene diamine was used as the spacer and, as the latex, "DRS-09" (manufactured by Nippon Gosei Gomu Kabushiki Kaisha, Japan; particle size: 5.1 μ ) was used.

(II) Preparation of A group substance-carrying latex

According to the same manner as described in Example 25 (II), the A group substance obtained in Example 1 (I) was supported on the spacer sensitized latex obtained in the above (I).

When anti A serum was added to this latex, agglutination reaction occurred. However, when anti B serum was used, no agglutination was observed.

## Example 30

### (I) Preparation of spacer sensitized latex

According to the same manner as described in Example 23 (I), a spacer sensitized latex was prepared except that hexamethylene diamine was used as the spacer and, as the latex, "Immutex G 0101" (manufactured by Nippon Gosei Gomu Kabushiki Kaisha, Japan; particle size: 0.7 $\mu$ ) was used.

### (II) Preparation of A group substance-carrying latex

According to the same manner as described in Example 23 (II), the A group substance obtained in Example 1 (I) was supported on the spacer sensitized latex obtained in the above (I).

When anti A serum was added to this latex, agglutination reaction occurred. However, when anti B serum was used, no agglutination was observed.

## Example 31

### (I) Preparation of spacer sensitized latex

According to the same manner as described in Example 25 (I), a spacer sensitized latex was prepared except that L-lysine was used as the spacer and, as the latex, "XP-5103" (manufactured by Nippon Gosei Gomu Kabushiki Kaisha, Japan; particle size: 3 $\mu$ ) was used.

### (II) Preparation of A group substance-carrying latex

According to the same manner as described in Example 25 (II), the A group substance obtained in Example 1 (I) was supported on the spacer sensitized latex obtained in the above (I).

When anti A serum was added to this latex, agglutination reaction occurred. However, when anti B serum was used, no agglutination was observed.

## Example 32

### (I) Preparation of spacer sensitized latex

According to the same manner as described in Example 25 (I), a spacer sensitized latex was prepared except that spermine was used as the spacer and, as the latex, "POLYBEAD" of fine particles having carboxyl groups on the surface thereof manufactured by Polyscience Inc., Warrington, U.S.A. (average particle size: 5.2 $\mu$ ; surface charge: 0.12 meqCOO$^-$/g) was used.

### (II) Preparation of A group substance-carrying latex

According to the same manner as described in Example 25 (II), the A group substance obtained in Example 1 (I) was supported on the spacer sensitized latex obtained in the above (I).

When anti A serum was added to this latex, agglutination reaction occurred. However, when anti B serum was used, no agglutination was observed.

## Example 33

Preparation of B group substance-carrying latex

The B group substance obtained in Example 7 (I) was supported on the spacer sensitized latex obtained in Example 23 (I) by using EDC to obtain the desired B group substance-carrying latex.

When anti B serum was added to this latex, agglutination reaction occurred. However, when anti A serum was used, no agglutination was observed.

## Example 34

Preparation of B group substance-carrying latex

The B group substance obtained in Example 7 (I) was supported on the spacer sensitized latex obtained in Example 25 (I) by using CMC to obtain the desired B group substance-carrying latex.

When anti B serum was added to this latex, agglutination reaction occurred. However, when anti A serum was used, no agglutination was observed.

## Example 35

Preparation of B group substance-carrying latex

The B group substance obtained in Example 7 (I) was supported on the spacer sensitized latex obtained in Example 26 (I) by using EDC to obtain the desired B group substance-carrying latex.

When anti B serum was added to this latex, agglutination reaction occurred. However, when anti A serum was used, no agglutination was observed.

## Example 36

Preparation of B group substance-carrying latex

The B group substance obtained in Example 7 (I) was supported on the spacer sensitized latex obtained in Example 27 (I) by using CMC to obtain the desired B group substance-carrying latex.

When anti B serum was added to this latex, agglutination reaction occurred. However, when anti A serum was used, no agglutination was observed.

## Example 37

Preparation of B group substance-carrying latex

The B group substance obtained in Example 7 (I) was supported on the spacer sensitized latex obtained in Example 27 (I) by using CMC to obtain the desired B group substance-carrying latex.

When anti B serum was added to this latex, agglutination reaction occurred. However, when anti A serum was used, no agglutination was observed.

## Experiment 3

Blood grouping according to slide method

According to the same manner as described above, blood grouping was carried out by using the A group substance-carrying latex obtained in Example 23 and the B group substance-carrying latex obtained in Example 33.

The results obtained from 25 sera are shown in Table 3.

Table 3

| Sera No. | Agglutination | | Judgment | Blood group test |
|---|---|---|---|---|
| | A group substance carrying latex | B group substance carrying latex | | |
| 1 | − | − | AB | AB |
| 2 | − | + | A | A |
| 3 | + | + | O | O |
| 4 | − | + | A | A |
| 5 | + | − | B | B |
| 6 | − | − | AB | AB |
| 7 | + | + | O | O |
| 8 | − | + | A | A |
| 9 | − | + | A | A |
| 10 | + | + | O | O |
| 11 | + | − | B | B |
| 12 | − | + | A | A |
| 13 | + | − | B | B |

## Table 3 (continued)

| Sera No. | Agglutination | | Judgment | Blood group test |
|----------|---------------|---------------|----------|-------------------|
| | A group substance carrying latex | B group substance carrying latex | | |
| 14 | − | + | A | A |
| 15 | + | + | O | O |
| 16 | − | + | A | A |
| 17 | + | + | O | O |
| 18 | + | − | B | B |
| 19 | − | + | A | A |
| 20 | + | + | O | O |
| 21 | + | − | B | B |
| 22 | + | + | O | O |
| 23 | − | − | AB | AB |
| 24 | − | + | A | A |
| 25 | − | + | A | A |

As seen from Table 3, the test results obtained by using the blood group substance-carrying latex of the present invention are completely corresponding to those of the blood group test.

Further, according to the same slide method as described above, blood grouping of various sera was carried out by using various combinations of the A group substance-carrying latexes obtained in Examples 24 to 32 and the B group substance-carrying latexes obtained in Examples 34 to 37. The results were completely corresponding to those of the blood group test.

The blood group substance-carrying latex of the present invention wherein the blood group substance is supported on the surface of the latex through chemical bonds has not been reported heretofore in the prior art and, therefore, it is a novel substance. And, in comparison with a conventional blood group substance-carrying latex prepared by a physical absorption method, the latex of the present invention can support a larger amount of the blood group substance because of chemical bonds and hardly causes removal of the substance. Accordingly, the blood group substance-carrying latex of the present invention shows very high agglutination activity to a serum to be tested. Particularly, when the blood group substance is supported through protein or spacers, clear agglutination image can be obtained because it is considered that the blood group substance would take such a steric configuration that it can be readily reacted with the corresponding agglutinin. Thereby, the latex of the present invention shows much higher agglutination activity to a serum to be tested in comparison with a conventional blood group substance-carrying latex. For example, human red blood cells show a highest agglutination activity and, in comparison with these, a conventional blood group substance-carrying latex shows only about 1/8 to 1/32 activity of red blood cells as expressed by a titer value. On the other hand, the latex of the present invention show about 1/2 to 1/4 activity of red blood cells as expressed by a titer value.

Further, in comparison with human red blood cells presently used in a "back typing test", the available period of the latex of the present invention is more than one year and it can be stored stably for that period, whereas the available period of human red blood cells is 3 to 4 weeks. In addition, the blood group substance-carrying latex of the present invention has the following advantages.

(1) Red blood cells cause hemolysis and, therefore, it is necessary to thoroughly wash them before use. However, in the latex of the present invention, such an operation is not required.

(2) The antigen activity of human red blood cells is varied. However, the blood group substance-carrying latex of the present invention has a constant group antigen activity to a serum to be tested and, therefore, its agglutination activity is also constant.

## Claims

1. A blood group substance-carrying latex which comprises latex particles and a blood group substance supported on the particles through chemical bonds.

2. The latex according to claim 1, wherein the blood group substance is supported on the particles through amide bonds.

3. The latex according to claim 1 or claim 2, wherein the latex particles have an average particle size of 0.01 to 20 $\mu$, and have carboxyl and/or amino groups on the surfaces thereof.

4. The latex according to any one of the preceding claims wherein the latex particles are protein sensitized latex particles.

5. The latex according to claim 4, wherein the blood group substance is supported on the protein sensitized latex particles by chemically binding the substance to the particles in the presence of a coupling agent.

6. The latex according to claim 4, wherein the blood group substance is supported on the protein sensitized latex particles through chromium ion.

7. The latex according to any one of claims 4-6, wherein the protein sensitized latex is that obtained by chemically binding protein to latex particles having functional groups selected from carboxyl group and amino group on the surfaces thereof in the presence of a coupling agent.

8. The latex according to any one of claims 4-6, wherein the protein sensitized latex is that obtained by physical adsorption of protein on latex particles.

9. The latex according to any one of claims 4-8, wherein the latex particles have an average particle size of 0.01 to 20 $\mu$.

10. The latex according to any one of claims 4-9, wherein the protein is bovine serum albumin or ovalbumin.

11. The latex according to claim 1, wherein the blood group substance is supported on the latexd particles through spacers.

12. The latex according to claim 11, wherein the latex particles have functional groups selected from the group consisting of carboxyl group and amino group on the surfaces thereof.

13. The latex according to claim 11 or claim 12, wherein the latex particles have an average particle size of 0.01 to 20 $\mu$.

14. The latex according to any one of claims 11-13, wherein the spacer is a material having functional groups selected from the group consisting of carboxyl group and amino group at the ends thereof.

15. The latex according to claim 14, wherein the spacer is a material selected from the group consisting of amino acids having 4 to 10 carbon atoms, alkylene diamines having 3 to 8 carbon atoms, polyalkylene polyamines having 8 to 12 carbon atoms, polyethylene polyamines having 4 to 6 carbon atoms and dicarboxylic acids having 9 to 14 carbon atoms.

16. A process for preparing a blood group substance-carrying latex which comprises reacting a latex with a blood group substance in the presence of a coupling agent to support the substance on the latex particles through amide bonds.

17. A process according to claim 16, wherein the latex particles have an average particle size of 0.01 to 20 $\mu$, and have carboxyl and/or amino groups on the surfaces thereof.

18. A process for preparing a blood group substance-carrying latex which comprises sensitizing protein to latex particles by means of chemical binding and/or physical adsorption to form a protein sensitized latex, and supporting the blood group substance on the protein sensitized latex.

19. A process for preparing a blood group substance-carrying latex which comprises binding a spacer material to latex particles in the presence of a coupling agent to form a spacer sensitized latex, and supporting the blood group substance on the spacer sensitized latex in the presence of a coupling agent.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 001 224 (F. HOFFMANN-LA ROCHE) * Abstract; page 8, lines 28-36; page 2, lines 24-32; Claims 3,4 * | 1-3 | G 01 N 33/80 G 01 N 33/546 |
| X | GB-A-2 027 031 (F. HOFFMANN-LA ROCHE) * Abstract; page 1, column 2, lines 65-122; page 3, column 1, lines 38-46; page 5, column 2, example 1; page 7, examples 11,12; claims 1,2,4,5,6,11,28,32 * | 1-5,7,9 ,10,12, 13,16- 18 | |
| X | EP-A-0 152 863 (F. HOFFMANN-LA ROCHE) * Claims 10,11; page 8, lines 6-17 * | 1 | |
| X | EP-A-0 134 660 (E.I. DU PONT DE NEMOURS) * Pages 13,14; page 20, lines 23-35; page 21, lines 1-23 * | 11,12, 15 | |
| X | EP-A-0 008 682 (F. HOFFMANN-LA ROCHE) * Claims 1-5; page 9, lines 17-25 * | 1-5,7,9 ,10 | |
| A | DE-A-3 022 278 (ORTHO DIAGNOSTICS) * Claims * | 1 | TECHNICAL FIELDS SEARCHED (Int. Cl.4) G 01 N |
| A | EP-A-0 106 685 (SYVA CO.) * Claims * | 1 | |

The present search report has been drawn up for all claims

| Place of search | Date of completion of the search | Examiner |
|---|---|---|
| THE HAGUE | 25-04-1988 | MEYLAERTS H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)